# EUROPÄISCHE PATENTSCHRIFT

(11) **EP 4 306 639 B1**
(45) Veröffentlichungstag und Bekanntmachung des Hinweises auf die Patenterteilung: **25.03.2026**
(21) Anmeldenummer: 21862177.9
(22) Anmeldetag: 05.08.2021
(51) Int. Cl.: C12N 15/113, A61K 35/14, A61K 35/22, A61P 35/00, A61K 9/50, C12N 15/11, C12N 15/88

(54) **VERFAHREN ZUM LADEN VON EXOSOMEN MIT KLEINEN NICHTCODIERENDEN RNAS**
METHOD FOR LOADING EXOSOMES WITH SMALL NON-CODING RNAS
PROCÉDÉ DE CHARGEMENT D'EXOSOMES AVEC DES ARN NON CODANTS

(30) Priorität: 27.08.2020 RU 2020128542
(43) Veröffentlichungstag der Anmeldung: 17.01.2024
(73) Patentinhaber: Gordeychuk, Vladimir Evgenevich, g. Krasnodar, 350001 (RU); Pospelov, Vadim Igorevich, Kolomna 141407 (RU); Rubtsov, Sergey Vladimirovich, Moscow 109316 (RU); Barsukov, Aleksandr Pavlovich, Rostov-na-Donu 344113 (RU)
(72) Erfinder: ABRAMOV, Aleksandr Andreevich, Moscow, 119634 (RU); PETKEVICH, Alisa Antonovna, Moscow, 109651 (RU); POSPELOV, Vadim Igorevich, Kolomna, 141407 (RU)
(74) Vertreter: Rentsch Partner AG
(86) Internationale Anmeldenummer: PCT/RU2021/000333
(87) Internationale Veröffentlichungsnummer: WO 2022/045927

(56) Entgegenhaltungen:
- WO-A1-2014/134132
- WO-A1-2014/134132
- CN-B- 105 779 586
- RU-C1- 2 548 816
- ТАМКОВИЧ С. Н., ТУТАНОВ О. С., ЛАКТИОНОВ П. П., TAMKOVICH S. N., TUTANOV O. S., LAKTIONOV P. P.: "Ekzosomy: mekhanizmy vozniknoveniia, sostav, transport, biologicheskaia aktivnost, ispolzovanie v diagnostike", BIOLOGICHESKIE MEMBRANY, vol. 33, no. 3, 2016, pages 163 - 175, XP055912379, DOI: 10.7868/S0233475516020122

## Beschreibung

Die Erfindung bezieht sich auf die Molekularbiologie, insbesondere auf Verfahren zum Beladen (Befüllen) von Exosomen mit kleiner nicht-kodierender RNA, um deren anschließende gezielte Abgabe an einzelne Gewebe lebender Organismen zu ermöglichen.

Es ist ein Verfahren zum Beladen von Exosomen mit Anthacyanidinen [Patent WO2014134132 Al Milk-derived microvesicle compositions and related methods] bekannt, bei dem die Exosomen aus Kuhmilch gewonnen werden, dann mit mikroRNA behandelt werden, was die Verwendung der Exosomen als mikroRNA-Transporter mit der Aussicht auf ihren Einsatz bei der Behandlung verschiedener Krankheiten ermöglicht. Diese Methode hat folgende Nachteile: 1) bei der bekannten Methode werden Exosomen aus Kuhmilch verwendet, wodurch es nicht möglich ist, eine hohe Effizienz bei der Abgabe an einzelne menschliche Gewebe zu erzielen. Aufgrund der unterschiedlichen Proteinsequenzen können Rinderexosomen im menschlichen Körper als fremdartig erkannt werden und im Vergleich zu menschlichen Exosomen in bestimmte Organe und Gewebe nicht effizient genug eindringen. 2) die bekannte Methode liefert keine Daten über die maximale Effizienz des Beladens von mikroRNA-Molekülen, und angesichts des Potenzials für weitere therapeutische Anwendungen und der recht hohen Kosten für die Synthese dieser Moleküle kann ein effizientes Beladen ein wesentlicher Faktor für die praktische Anwendung von in Exosomen geladener mikroRNA sein.

Der technische Effekt, der durch die Durchführung des beanspruchten Verfahrens erreicht wird, ist die Möglichkeit eines maximalen Beladens der Exosomen, die aus biologischen Flüssigkeiten des Menschen erhalten werden, mit kleinen nicht-kodierenden mikroRNAs, was es in Zukunft ermöglichen wird, beladene Exosome als Transportstrukturen zu nutzen, um therapeutische Wirkstoffe zu Tumorzellen in lebenden Organismen zu bringen.

Das oben genannte technische Ergebnis wird dadurch erzielt, dass eine phosphatgepufferte Kochsalzlösung (PBS) mit Exosomen aus menschlichem Urin oder menschlichem peripheren Venenblut und kleine nicht-kodierende RNA in einem Verhältnis von 1 ml Lösung mit 1*10^10 bis 5*10^10 Exosomen und 10*10^12 bis 50*10^12 Kopien kleiner nicht-kodierender RNA gemischt wird, dann die erhaltene Mischung im Thermostat bei 35-40 °C für 14-18 Stunden belassen wird.

Nach der Inkubation der Substanzen ist ein Ultrazentrifugationsschritt erforderlich, um die Exosomen mit der beladenen Substanz - während der Ultrazentrifugation bilden sie ein Sediment am Boden des Röhrchens - von der beladen-freien Substanz zu trennen, die bei der Ultrazentrifugation in der Lösung bleibt (Überstand).

Die Ultrazentrifugation wird vorzugsweise bei 100000 g für 2 Stunden durchgeführt, um eine maximale Ausfällung der beladenen Exosomen in der Lösung zu gewährleisten.

### Beispiel 1

Vorher durch die Methode der gradienten Ultrazentrifugation an Saccharose aus menschlichem Urin in der Menge von 100 ml erhaltene Exosome im Umfang von 10*10^10 und 80*10^12 Kopien von kleiner nicht-kodierender RNA hsa-mir-34a wurden in 8 ml PBS gemischt. Die erhaltene Mischung wurde in 8 1-ml-Proben aufgeteilt: die erste Probe wurde 10 Stunden lang im Thermostat bei 40 °C, die zweite Probe wurde 12 Stunden lang im Thermostat bei 40 °C belassen, die dritte Probe wurde 14 Stunden lang im Thermostat bei 40 °C belassen, die vierte Probe wurde 16 Stunden lang im Thermostat bei 40 °C belassen und die fünfte Probe wurde 18 Stunden lang im Thermostat bei 40 °C belassen, die sechste Probe wurde 20 Stunden lang bei 40 °C im Thermostat belassen, die siebte Probe wurde 22 Stunden lang bei 40 °C im Thermostat belassen und die achte Probe wurde 24 Stunden lang bei 40 °C im Thermostat belassen. Nach Ablauf der entsprechenden Inkubationszeiten wurde 2 Stunden lang eine Ultrazentrifugation der Probe bei 100000 g vorgenommen, danach wurde das entstandene Präzipitat in 1 ml PBS verdünnt, und es wurden drei Gefrier-Auftau-Zyklen durchgeführt, um die Exosomen zu zerstören und das freie Markermolekül (nicht-kodierende RNA has-mir-34a) zu erhalten, gefolgt von einer PCR-Analyse in Echtzeit. Das Ergebnis ist, dass die Konzentration der kleinen nicht-kodierenden RNA hsa-mir-34a in den Proben 3, 4 und 5 am höchsten war. Tabelle 1 zeigt deutlich, dass die Konzentration der geladenen mikroRNA gleichmäßig ansteigt und in der dritten, vierten und fünften Probe ihren Höchstwert erreicht, gefolgt von einem gleichmäßigen Rückgang, was auf die effizienteste Inkubationszeit zwischen 14 und 18 Stunden hinweist.

**Tabelle 1: Auswirkung der Inkubationszeit auf die Effizienz der mikroRNA-Beladung von Exosomen.**

| Probe und Inkubati onszeit | Nº1 | Nº2 | Nº3 | Nº4 | Nº5 | Nº6 | Nº7 | Nº8 |
|---|---|---|---|---|---|---|---|---|
| | 10h | 12h | 14h | 16h | 18h | 20h | 22h | 24h |
| Konzentration an kleiner nichtkodieren der RNA hsa-mir-34a (Kopien/ ml) | 1,89 | 2,12 | 2,84 | 2,97 | 2,87 | 2,51 | 2,38 | 2,05 |
| | *10^ 12 | *10^ 12 | *10^ 12 | *10^ 12 | *10^ 12 | *10^ 12 | *10^ 12 | *10^ 12 |

### Beispiel 2

Vorher durch die Methode der gradienten Ultrazentrifugation an Saccharose aus menschlichem Urin in der Menge von 100 ml erhaltene Exosome im Umfang von 2*10^9 und 10*10^12 Kopien von kleiner nicht-kodierender RNA hsa-mir-122 wurden in 1 ml PBS gemischt (Probe Nr.1). Es wurden auch Proben mit einer Exosomenkonzentration von 1*10^10 (Probe Nr.2), mit einer Exosomenkonzentration von 5*10^10 (Probe Nr.3) und mit einer Exosomenkonzentration von 25*10^10 (Probe Nr.4) erhalten. Die erhaltenen Proben wurden 16 Stunden lang bei 40 °C im Thermostat belassen. Nach der entsprechenden Inkubationszeit wurden die Proben 2 Stunden lang bei 100000 g ultrazentrifugiert, dann wurde das erhaltene Präzipitat in 1 ml PBS verdünnt, und es wurden drei Gefrier-Auftau-Zyklen durchgeführt, um die Exosomen zu zerstören und das freie Markermolekül (kleine nicht-kodierende RNA hsa-mir-122) zu erhalten, gefolgt von einer PCR-Analyse in Echtzeit. Infolgedessen war die Konzentration an kleiner nicht-kodierender RNA hsa-mir-122 in der 2. und 3. Probe am höchsten. Tabelle 2 zeigt deutlich, dass die Konzentration der geladenen mikroRNA gleichmäßig ansteigt und in der 2. und 3. Probe ein Maximum erreicht, gefolgt von einem gleichmäßigen Rückgang, was auf eine optimale Konzentration der Exosomen im Bereich von 1*10^10/ml bis 5*10^10/ml hinweist.

**Tabelle 2: Auswirkung der Exosomenkonzentration auf die Effizienz der mikroRNA-Beladung**

| Probe und Exosomenkonzentration | Nº1 | Nº2 | Nº3 | Nº4 |
|---|---|---|---|---|
| | 2*10^9/ml | 1*10^10/ml | 5*10^10/ml | 25*10^10/ml |
| Konzentration an kleiner nicht-kodierender RNA hsa-mir-122 (Kopien/ml) | 1,37 | 2,79 | 2, 97 | 2,24 |
| | *10^12 | *10^12 | *10^12 | *10^12 |

### Beispiel 3

Vorher durch die Methode der gradienten Ultrazentrifugation an Saccharose aus menschlichem Urin in der Menge von 100 ml erhaltene Exosome im Umfang von 30*10^10 und 60*10^12 Kopien von kleiner nicht-kodierender RNA hsa-mir-16 wurden in 6 ml PBS gemischt. Die erhaltene Mischung wurde in 6 1-ml-Proben aufgeteilt: die erste Probe wurde 16 Stunden lang im Thermostat bei 25 °C belassen, die zweite Probe wurde 16 Stunden lang im Thermostat bei 30 °C belassen, die dritte Probe wurde 16 Stunden lang im Thermostat bei 35 °C belassen, die vierte Probe wurde 16 Stunden lang im Thermostat bei 40 °C belassen, die fünfte Probe wurde 16 Stunden lang im Thermostat bei 45 °C für 16 Stunden belassen und die sechste Probe wurde 16 Stunden lang im Thermostat bei 50 °C belassen. Nach der entsprechenden Inkubationszeit wurde die Probe 2 Stunden lang bei 100000 g ultrazentrifugiert. Anschließend wurde das entstandene Präzipitat in 1 ml PBS verdünnt, wurden drei Gefrier-Auftau-Zyklen durchgeführt, um die Exosomen zu zerstören und das freie Markermolekül zu erhalten (kleine nicht-kodierende RNA has-mir-16), gefolgt von einer PCR-Analyse in Echtzeit. Infolgedessen war die Konzentration an kleiner nicht-kodierender RNA hsa-mir-16 in der dritten und vierten Probe am höchsten. Tabelle 3 zeigt deutlich, dass die Konzentration der geladenen mikroRNA gleichmäßig ansteigt und in der 3. und 4. Probe ein Maximum erreicht, gefolgt von einem gleichmäßigen Rückgang, was darauf hindeutet, dass die effektivste Inkubationstemperatur im Bereich von 35-40 °C liegt.

**Tabelle 3: Einfluss der Inkubationstemperatur auf die Effizienz der mikroRNA-Beladung von Exosomen.**

| Probe und Inkubation szeit | Nº1 | Nº2 | Nº3 | Nº4 | Nº5 | Nº6 |
|---|---|---|---|---|---|---|
| | 25°C | 30 °C | 35 °C | 40 °C | 45 °C | 50 °C |
| Konzentration an kleiner nicht-kodierende r RNA hsa-mir-16 (Kopien/ml ) | 0,78 | 1,88 | 2,79 | 2,84 | 2,11 | 1,91 |
| | *10^12 | *10^12 | *10^12 | *10^12 | *10^12 | *10^12 |

### Beispiel 4

Vorher durch die Methode der gradienten Ultrazentrifugation an Saccharose aus menschlichem Urin in der Menge von 100 ml erhaltene Exosome im Umfang von 5*10^10 und 1*10^12 Kopien von kleiner nicht-kodierender RNA hsa-mir-122 wurden in 1 ml PBS gemischt (Probe Nr.1). Es wurden auch Proben mit einer Konzentration an kleiner nicht-kodierender RNA von 10*10^12 Kopien (Probe Nr.2), einer Konzentration an kleiner nicht-kodierender RNA von 50*10^10 Kopien (Probe Nr.3) und einer Konzentration an kleiner nicht-kodierender RNA von 100*10^12 Kopien (Probe Nr.4) gewonnen. Die erhaltenen Proben wurden 16 Stunden lang im Thermostat bei 40 °C belassen. Nach einer entsprechenden Inkubationszeit wurden die Proben 2 Stunden lang bei 100000 g ultrazentrifugiert. Anschließend wurde das erhaltene Präzipitat in 1 ml PBS verdünnt und es wurden 3 Gefrier-Auftau-Zyklen durchgeführt, um die Exosomen zu zerstören und das freie Markermolekül (kleine nicht-kodierende RNA hsa-mir-122) zu erhalten, gefolgt von PCR-Analyse in Echtzeit. Infolgedessen war der Prozentsatz an in die Exosome geladener kleiner nicht-kodierender RNA hsa-mir-122 in der zweiten und dritten Probe am höchsten. Tabelle 4 zeigt deutlich, dass der Prozentsatz an geladener mikroRNA gleichmäßig ansteigt und in der 2. und 3. Probe ein Maximum erreicht, gefolgt von einem gleichmäßigen Rückgang, was darauf hindeutet, dass die optimale Konzentration an kleiner nicht-kodierender RNA im Bereich von 10*10^12/ml bis 50*10^12/ml liegt.

**Tabelle 4: Auswirkung der Konzentration an kleiner nicht-kodierender mikroRNA auf die Effizienz der Beladung**

| Probe und Konzentration an kleiner nicht-kodierender RNA | Nr. 1 | Nr.2 | Nr.3 | Nr. 4 |
|---|---|---|---|---|
| | 1*10^12 /ml | 10*10^12 /ml | 50*10^12 /ml | 100*10^12 /ml |
| % von in die Exosomen geladener kleiner nicht-kodierender RNA hsa-mir-122 | 25% | 28% | 30% | 16% |

Es ist darauf hinzuweisen, dass jede Art von kleiner nicht-kodierender RNA mit beliebiger Nukleotidsequenz verwendet werden kann, ohne dass dies die Qualität der Beladung beeinträchtigt, und dass die spezifische mikroRNA nur auf der Grundlage der Zielsetzung ausgewählt wird.

## Patentansprüche

1. Verfahren zum Beladen von Exosomen mit kleiner nicht-kodierender RNA, bei dem Exosomen, die aus Urin oder peripherem Venenblut des Menschen erhalten wurden, in phosphatgepufferter Kochsalzlösung (PBS) mit kleiner nicht-kodierender RNA im Verhältnis 1 ml Lösung von 1*10^10 bis 5*10^10 Exosomen und 10*10^12 bis 50*10^12 Kopien kleiner nicht-kodierender RNA gemischt werden, dann die Mischung bei 35-40 °C für 14-18 Stunden im Thermostat belassen wird.

## Claims

1. Method for loading exosomes with small non-coding RNA, in which exosomes obtained from human urine or peripheral venous blood are mixed in phosphate-buffered saline (PBS) with small non-coding RNA in a ratio of 1 ml of solution of 1*10^10 to 5*10^10 exosomes and 10*10^12 to 50*10^12 copies of small non-coding RNA, then the mixture is left in the thermostat at 35-40 °C for 14-18 hours.

## Revendications

1. Procédé pour charger des exosomes avec de petits ARN non codants, dans lequel des exosomes obtenus à partir d'urine ou de sang veineux périphérique humain sont placés dans une solution saline tamponnée au phosphate (PBS) avec de petits ARN non codant dans un rapport de 1 ml de solution de 1*10^10 à 5*10^10 exosomes et 10*10^12 à 50*10^12 copies de petits ARN non codants, puis le mélange est laissé dans un thermostat à 35-40 °C pendant 14-18 heures.
